# EUROPEAN PATENT APPLICATION

(11) **EP 2 742 894 A1**
(43) Date of publication of application: **18.06.2014**
(21) Application number: 13194932.3
(22) Date of filing: 28.11.2013
(51) Int. Cl.: A61B 19/00

(54) **Use of physician eye tracking during a procedure**

(30) Priority: 11.12.2012 US 201213710848
(71) Applicant: Biosense Webster (Israel), Ltd., Yokneam 20692 (IL)
(72) Inventor: Katz, Natan Sharon, 27000 Kiryat Bialik (IL); Krupnik, Ronen, 21681 Karmiel (IL); Turgeman, Aharon, 30900 Zichron Ya'acov (IL); Cohn, Goren, 34670 Haifa (IL); Zilberman, Israel, 20692 Yokneam (IL)
(74) Representative: Carpmaels & Ransford LLP

(57) **Abstract**

A method for displaying information, including presenting on one or more monitors a plurality of distinct elements in a first spatial relationship with one another, the plurality of distinct elements being illustrative of a state of a medical procedure. The method also includes measuring gaze directions, towards the plurality of the elements, of an implementer of the medical procedure while the procedure is being performed. In response to the measured gaze directions, the plurality of distinct elements are rearranged on the one or more monitors into a second spatial relationship with one another.

## Description

### FIELD OF THE INVENTION

The present invention relates generally to arrangement of data on one or more monitors during a medical procedure, and specifically to the use of eye tracking to facilitate the arrangement of the data.

### BACKGROUND OF THE INVENTION

The amount of data presented to a professional performing a medical procedure on a subject may be extremely large. Especially during performance of the procedure, the large amount of data presented may even be counter-productive, possibly unnecessarily complicating the task of the professional. Any system which reduces the complications involved with presentation of large amounts of data would be advantageous.

Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### SUMMARY OF THE INVENTION

An embodiment of the present invention provides a method for displaying information, including:
presenting on one or more monitors a plurality of distinct elements in a first spatial relationship with one another, the plurality of distinct elements being illustrative of a state of a medical procedure;
measuring gaze directions, towards the plurality of the elements, of an implementer of the medical procedure while the procedure is being performed; and
in response to the measured gaze directions, rearranging on the one or more monitors the plurality of distinct elements into a second spatial relationship with one another.

Typically, the one or more monitors include two or more monitors.

In a disclosed embodiment, rearranging the plurality of distinct elements includes the implementer selecting, using gaze direction, a given distinct element, and further selecting, using gaze direction, a new location for the given distinct element on the one or more monitors.

In a further disclosed embodiment the method further includes presenting the second spatial relationship on the one or more monitors prior to a subsequent medical procedure being performed by the implementer.

In a yet further disclosed embodiment the method further includes classifying the distinct elements as movable or fixed distinct elements, and rearranging the plurality of distinct elements includes relocating at least one of the movable elements while maintaining locations of the fixed elements on the one or more monitors.

In an alternative embodiment, the state of the medical procedure includes a multiplicity of sub-states of the medical procedure, and presenting the plurality of distinct elements includes presenting the elements during a given sub-state, and measuring the gaze directions includes measuring the gaze directions during the given sub-state, and rearranging the plurality of distinct elements includes rearranging the elements for the given sub-state. Typically, the alternative embodiment also includes defining the sub-states in response to parameters of equipment used during the medical procedure.

In a further alternative embodiment measuring the gaze directions towards the plurality of the elements includes registering conscious observation of at least one of the elements. Registering conscious observation of the at least one of the elements may include determining that a time period of a gaze direction towards the at least one of the elements exceeds a preset time. Alternatively or additionally, registering conscious observation of the at least one of the elements includes determining that a number of observations of the at least one of the elements exceeds a preset number.

There is further provided, according to an embodiment of the present invention, apparatus for displaying information, including:
one or more monitors configured to present a plurality of distinct elements in a first spatial relationship with one another, the plurality of distinct elements being illustrative of a state of a medical procedure;
an eye tracker, configured to measure gaze directions towards the plurality of the elements of an implementer of the medical procedure while the procedure is being performed; and
a processor which is configured, in response to the measured gaze directions, to rearrange on the one or more monitors the plurality of distinct elements into a second spatial relationship with one another.

The present disclosure will be more fully understood from the following detailed description of the embodiments thereof, taken together with the drawings, in which:

### BRIEF DESCRIPTION OF THE DRAWINGS

Fig. 1 is a schematic illustration of an eye-tracking information presentation system, according to an embodiment of the present invention;
Fig. 2 is a schematic diagram illustrating different distinct elements presented on monitors used in the system of Fig. 1, according to an embodiment of the present invention;
Fig. 3 is a flowchart describing steps taken during operation of the system, according to an embodiment of the present invention;
Fig. 4 illustrates the generation of a new spatial relationship of displayed elements using the flowchart, according to an embodiment of the present invention;
Fig. 5 is a flowchart describing steps taken during operation of the system of Fig. 1, according to an alternative embodiment of the present invention; and
Fig. 6 illustrates presentation of a new spatial relationship of displayed elements, according to an embodiment of the present invention.

### DETAILED DESCRIPTION OF EMBODIMENTS

### OVERVIEW

An embodiment of the present invention provides a method for improving the display of information to a professional conducting a medical procedure. Initially information representative of the procedure, such as tables, graphs, three-dimensional (3D) representations of an organ being operated on, and/or characteristics of the organ, is displayed on one or more monitors. The information is assumed to be in the form of distinct elements (such as the examples given above) which are arranged on the monitors in an initial spatial relationship.

During the procedure, the gaze direction of the professional towards the elements is measured. The professional is herein also termed the procedure implementer, or just the implementer. Typically, the measured gaze directions are evaluated to determine if the implementer appears to be consciously observing a particular element, and a processor records these elements.

Using the record of the observed elements, the processor may rearrange the elements into an alternative spatial relationship. Typically, the rearrangement is implemented by invoking the gaze direction of the implementer to select a given element for relocation on the monitors, as well as to select a new location on the monitors for the given element.

### SYSTEM DESCRIPTION

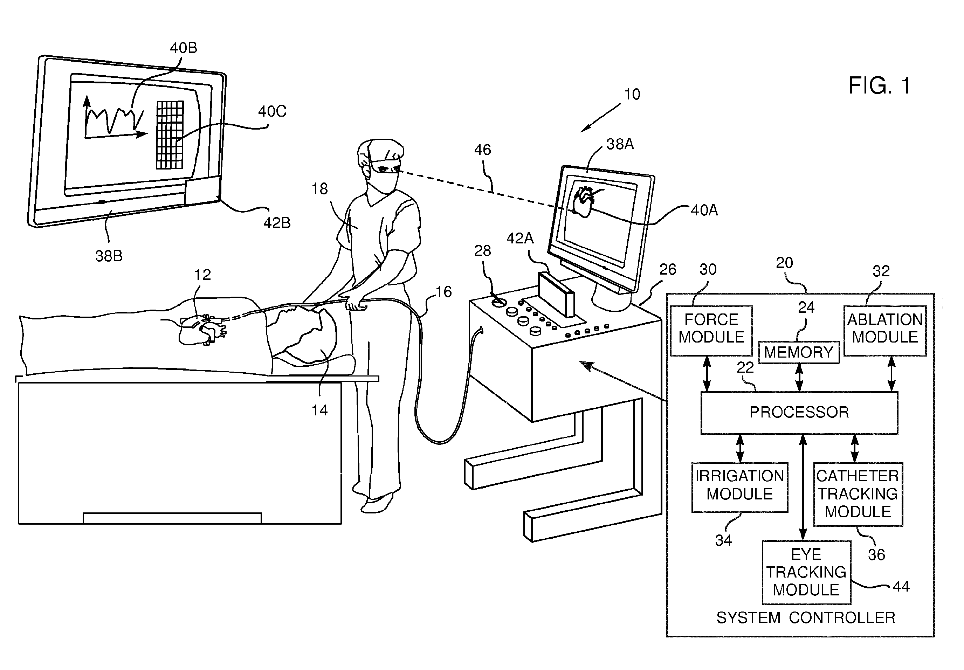

Reference is now made to Fig. 1, which is a schematic illustration of an eye-tracking information presentation system 10, according to an embodiment of the present invention. System 10 is used to control the presentation of information on one or more display monitors that are used during a medical procedure performed by a user of the system. By way of example, in the following description system 10 is assumed to be used during an ablation procedure that is performed on a heart 12 of a subject 14. The procedure is performed by insertion of a probe 16 into the body of subject 14, and a user 18 of system 10 is herein by way of example assumed to be a medical professional who implements the procedure.

System 10 may be controlled by a system controller 20, comprising a processor 22 communicating with a memory 24. Controller 20 is typically mounted in a console 26, which comprises operating controls that typically include a pointing device 28 such as a mouse or trackball, that professional 18 uses to interact with the processor. The processor uses software stored in memory 24, to operate system 10. The software stored in memory 24 typically includes a force module 30, an ablation module 32, an irrigation module 34, and a magnetic field catheter-tracking module 36. U.S. Patent Application Publications, 2009/0138007, 2011/0224664, and 2012/0157890, whose disclosures are incorporated herein by reference, describe means for performing the functions of these modules. For clarity in the figure the modules are shown separate from memory 24, and the functions of the modules are explained below.

The software may be downloaded to processor 22 in electronic form, over a network, for example, or it may, alternatively or additionally, be provided and/or stored on non-transitory tangible media, such as magnetic, optical, or electronic memory.

Results of the operations performed by processor 22 are presented to the professional on one or more display monitors 38A, 38B, ... , which are collectively referred to herein as monitors 38. Monitors 38 typically present distinct elements 40 which are illustrative of a state of a medical procedure that is implemented by professional 18, and the monitors may be located in any positions that are convenient for viewing by professional 18. By way of example monitor 38A is assumed to be mounted on console 26, and monitor 38B is assumed to be mounted above subject 14.

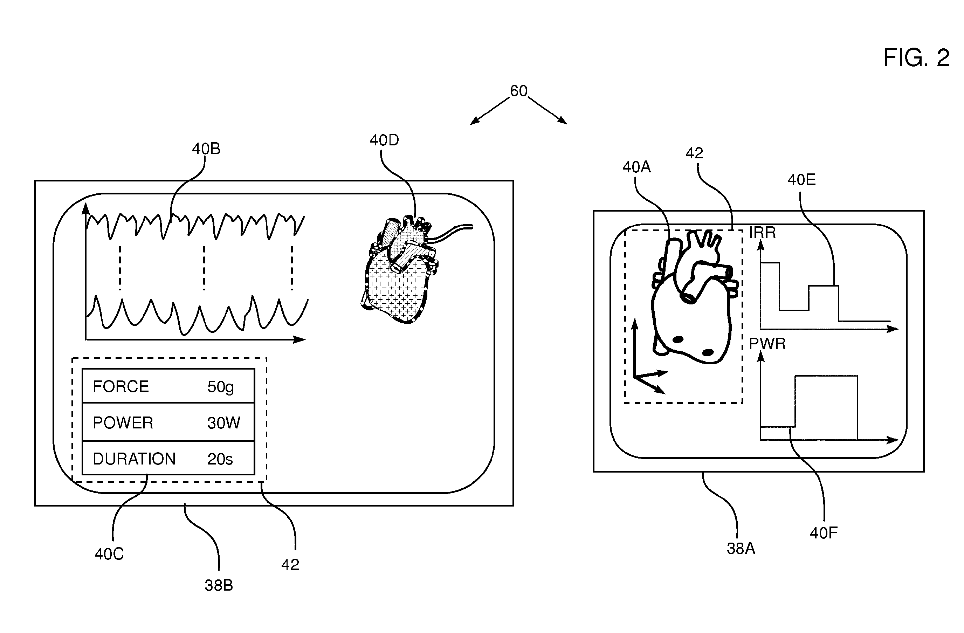

As necessary, in the description herein distinct elements 40 are differentiated by appending a letter A, B, ... to the identifying numeral 40. A typical example of a distinct element comprises a three-dimensional (3D) representation 40A of heart 12, and representation 40A may also indicate a location of a distal end of probe 16. Other examples of distinct elements include voltage vs. time graphs 40B of ECG signals generated by subject 14, and an ablation table 40C illustrating parameters regarding the ablation procedure, such as the force applied by the distal end of the probe, and the power used for the ablation. Other examples of distinct elements presented on the display monitors are described with reference to Fig. 2 below.

System 10 operates by tracking the gaze direction of professional 18 at periods when the professional is looking at one of monitors 38. A number of eye-tracking devices are known in the art. Such devices include systems that track the eye-viewing direction relative to the direction of the head of professional 18, so requiring tracking of the head of the professional, including spectacle-based systems worn by the professional. Alternatively, other devices may track the gaze direction by measuring a direction of viewing of the eye relative to an external fiduciary, which typically projects an infra-red beam to the eyes of professional 18. Other systems, such as measuring changes in skin potentials in proximity to the eye, may also be used. In embodiments of the present invention any convenient method may be used to track the gaze direction of professional 18 with respect to monitors 40, and all such methods are assumed to be comprised within the scope of the present invention.

For simplicity, in the following description, each monitor 40 is assumed to be associated with a respective eye-tracking device. Thus, a first eye-tracking device 42A is mounted on console 26, so that it is in proximity to monitor 38A, and a second eye-tracking device 42B is mounted on the frame of monitor 38B.

Controller 20 comprises an eye-tracking module 44, which receives data from devices 42A and 42B, by wireless or by one or more cables, and which uses the data to provide processor 22 with a gaze direction 46 of professional 18.

Fig. 2 is a schematic diagram illustrating different distinct elements 40 presented on monitors 38, according to an embodiment of the present invention. Monitor 38A displays 3D image 40A of heart 12 (referred to above), an irrigation flow rate vs. time graph 40E, and an ablation power vs. time graph 40F. Monitor 38B displays ECG graphs 40B and ablation table 40C, also referred to above. In addition, monitor 38B displays another representation 40D of heart 12, which is assumed to be different from representation 40A. For example, representation 40D may illustrate local activation times (LATs) associated with heart 12 in a color format, while representation 40A may illustrate positions within the heart that have been ablated.

As described in more detail below, each distinct element 40 may be classified as being fixed or movable. A fixed element maintains its location within a given monitor 38. A movable element may be translated within its monitor, or may be transferred to another monitor. By way of example, in Fig. 2 elements which are movable have been enclosed in broken rectangles 42, i.e., table 40C and representation 40A, and elements which are not so enclosed are assumed to be fixed.

During a medical procedure performed while system 10 is operating, there are a number of different states or phases of the procedure, typically starting with an initial preparatory phase and concluding with a final "wrap-up" phase. Between the bounding initial and final phases there are a number of intermediate phases, or sub-states, of the procedure.

Actions performed in each of the intermediate sub-states, as well as the bounding phases, depend on a number of factors. Table I below lists typical factors that influence the actions, and gives examples of these factors.

**Table I**

| **Factors influencing actions performed during a medical procedure** | **Examples of Factors** |
|---|---|
| Type of procedure | • Ablation of heart to correct |
| | atrial tachycardia. |
| | • Investigation of heart to quantify electrophysiological parameters. |
| | • Exploratory investigation of bladder |
| Equipment used during the procedure | • Multi-electrode catheter |
| | • Catheter providing contact force measurement |
| | • Catheter using irrigation |
| | • Catheter tracker |
| | • ECG recorder |
| | • Rigid Endoscope |
| | • Flexible Endoscope |
| | • X-ray machine |
| | • MRI machine |
| Characteristics of subject | • Male, aged 55 |
| | • Female, aged 75 |
| Professional performing procedure | • Physician A |
| | • Physician B |

Those having ordinary skill in the art will be able to list factors other than those listed in Table I that influence actions performed during a given medical procedure, and all such factors are assumed to be comprised within the scope of the present invention.

As stated above, there are a number of sub-states that occur during performance of a medical procedure. For clarity and simplicity, in a Table II below giving examples of sub-states, the medical procedure performed is assumed to be an ablation procedure to correct atrial tachycardia.

**Table II**

| **Sub-State** | **Actions Performed during Sub-State** |
|---|---|
| Initial insertion of catheter towards heart | • Tracking of distal end of catheter |
| Entry of catheter into heart (no ablation, no irrigation) | • Tracking of distal end of catheter |
| | • Measurement of electric potentials of endocardium |
| Ablation of endocardium (no irrigation) | • Tracking of distal end of catheter |
| | • Measurement of electric potentials of endocardium |
| | • Measurement of power delivered during ablation |
| | • Measurement of contact force applied by catheter |
| Irrigation of endocardium (no ablation) | • Tracking of distal end of catheter |
| | • Measurement of irrigation flow rate |
| Ablation with irrigation | • Tracking of distal end of catheter |
| | • Measurement of power delivered during ablation |
| | • Measurement of contact force applied by catheter |
| | • Measurement of irrigation flow rate |
| Removal of catheter from heart and from subject | • Tracking of distal end of catheter |

It will be understood that at least some of the sub-states may repeat. For example, in Table II the sub-states of ablation without irrigation and irrigation without ablation may repeat in a sequential manner. It will also be understood that the sub-states for any particular medical procedure are characteristic of the procedure, and that those having ordinary skill in the medical procedure arts will be able to itemize the sub-states for a given procedure, and to list actions performed during each of the sub-states.

Typically, the sub-states for a given medical procedure may be differentiated by different physical parameters of equipment used for the procedure, and processor 22 may use measures of the parameters to identify and define a sub-state of a procedure. Such physical parameters comprise, for example, the location and orientation of the distal end of the catheter used for the procedure, whether ablation is being applied, and if it is applied the power being used, whether irrigation is being used and the rate of flow of irrigating fluid, and the value of the contact force impressed on the catheter.

In addition to identifying and defining sub-states of a medical procedure using physical parameters of equipment used, processor 22 may use at least some of the factors (influencing actions performed during a medical procedure) exemplified in Table I to further identify and define sub-states of a procedure. For example, if an X-ray machine and a magnetic field catheter-tracking module 36 (Fig. 1) are both used to track a catheter, then in Table II each of the sub-states (which all use tracking of the catheter distal end) may be further sub-divided into three sub-sub-states: only module 36 is used; only the X-ray machine is used; and both module 36 and the X-ray machine are used.

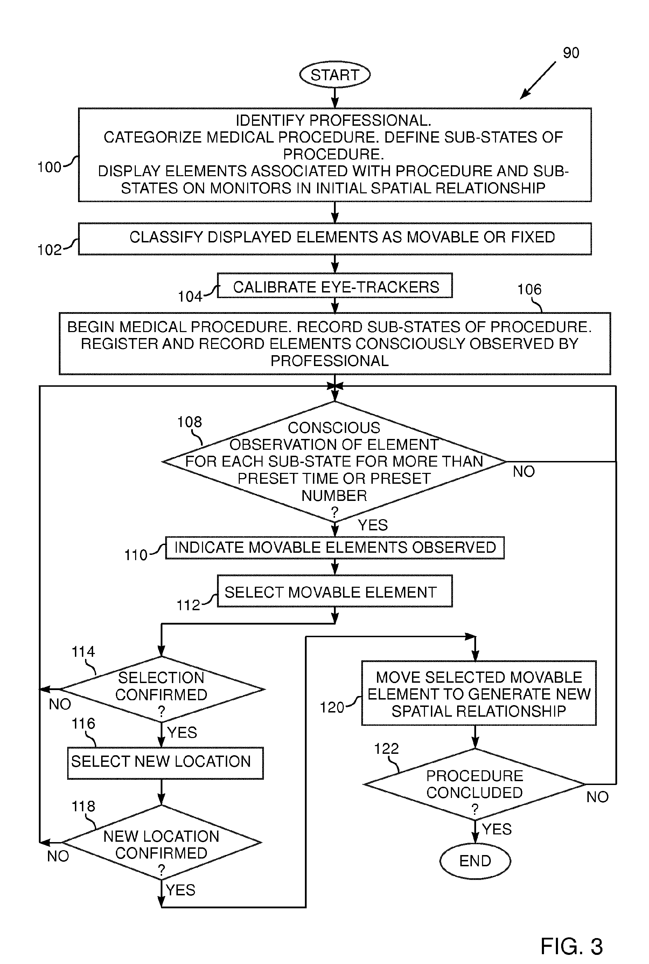

Fig. 3 is a flowchart 90 describing steps taken during operation of system 10, according to an embodiment of the present invention. The steps of the flowchart are under overall control of processor 22. In an initial step 100, professional 18 performing the medical procedure identifies him or herself to processor 22, and categorizes the procedure. Once the procedure has been categorized, processor 22 defines the sub-states that may be used during the medical procedure. Definition of the sub-states typically uses entities exemplified in Tables I and II above, and may be accomplished interactively with professional 18, for instance by the professional identifying which equipment is to be used during the categorized procedure.

The processor also displays distinct elements 40 associated with the procedure and the sub-states on monitors 38, the elements having a preset spatial relationship with one another, such as is exemplified in relationship 60 (Fig. 2). The preset spatial relationship may have been provided to processor 22 by an installer of system 10 or by another operator, such as a prior user 18, of the system; alternatively, the preset spatial relationship may have been provided by professional 18 in a previous use of the system. Such a method for providing the preset spatial relationship is described below.

In an element classification step 102, professional 18 initially classifies distinct elements 40 on monitors 38 as movable or fixed. The initial classification is typically performed by the professional selecting specific elements 40 using pointing device 28. Referring back to Fig. 2, table 40C and image 40A have been selected to be movable, as illustrated by broken rectangles 42, and the other distinct elements 40 have been selected to be fixed.

In an eye-tracking calibration step 104 processor 22 calibrates eye-tracking devices 42A and 42B for the professional using system 10. Typically, eye-tracking module 44 projects calibration patterns, such as highlights for specific elements 40, onto monitors 38A and 38B, and the eye-tracking module acquires signals from devices 42A and 42B while the professional observes the calibration patterns. The module processes the signals to determine a gaze direction, and processor 22 correlates the gaze direction with the projected calibration patterns.

In a procedure performance step 106, professional 18 begins a medical procedure. During the procedure, processor 22 records in which sub-state the procedure is in, and tracks the gaze direction of the professional. Processor 22 uses the measured gaze directions to register and record which distinct element 40 the professional is consciously observing.

Typically, the processor assumes that conscious observation of a given distinct element is occurring if the gaze direction towards the element occurs for greater than a preset time period. The preset time period may be of the order of 1 second or more. Conscious observation of a given element may also be assumed if the element is gazed at for an alternative preset time period, interspersed with glances at another region, or during observation of a number of regions in sequence. The processor determines if conscious observation has been made using a location stability algorithm, which incorporates the preset time periods above, as well as allowed variations from the periods (e.g., a preset number of standard deviations from the preset periods).

In some embodiments a value of the preset time period and/or of the alternative preset time period may be determined during eye-tracking calibration step 104, for example by projecting two or more calibration patterns simultaneously or sequentially on a monitor and asking the professional to observe the patterns.

In a comparison step 108, processor 22 determines if one or more of movable elements 40, that have been identified in step 102, have been consciously observed for more than a preset number of times, or for more than a preset time period. A typical preset number of times is 10; a typical preset time period is 10s. Processor 22 performs the comparison for each different sub-state of the procedure.

If in step 108 neither the preset number nor the preset time period has been exceeded, the flow chart returns to step 106 and continues to record the professional gaze direction and the sub-state of the medical procedure.

If in step 108 either the preset number or the preset time period has been exceeded, i.e., the comparison provides a positive return, in a continuation step 110 the processor indicates to the professional that the preset value has been exceeded for one or more selected movable elements 40. Typically, the processor may place a notice on one or both monitors 38A, 38B of the positive return. The processor indicates which movable elements 40 have been selected, for example by highlighting appropriate movable elements 40; the processor also configures the selected movable elements so that they may be moved, as described in the following step; in addition, typically in the notice referred to above, the processor indicates that the professional may alter the locations, on monitors 38A or 38B, of the selected movable elements.

In an element selection step 112, the professional uses his or her gaze direction to select one of the movable elements indicated in step 110. Typically, the processor uses a comparison 114 for initial confirmation from the professional that the element has been selected. Such confirmation may comprise any convenient indication from the professional, such as gazing, for the preset time period referred to above, at a selected movable element to be relocated. Assuming that the initial confirmation has been provided, in a new location step 116 the professional chooses, using gaze direction, a new location for the selected movable element. The processor uses a comparison 118, similar to comparison 114, for subsequent confirmation of the new location, i.e., by gazing for the preset time period at the chosen new location on one of monitors 38.

If either of comparisons 114 or 118 returns a negative value, the flowchart returns to comparison step 108.

In a relocation step 120, on receipt of confirmation of the new location by a positive return to comparison 118, the processor relocates selected movable element 40 from its initial location to its new location. The relocation generates a new spatial relationship for elements 40.

In a final comparison step 122 the processor checks if the procedure initiated in step 106 has concluded. If it has concluded the flowchart ends. If the procedure has not concluded, the flowchart returns to comparison step 108.

In some embodiments the new spatial relationship generated in step 120 of the flowchart is used as a default initial spatial relationship of elements 40 in a subsequent medical procedure performed by the professional.

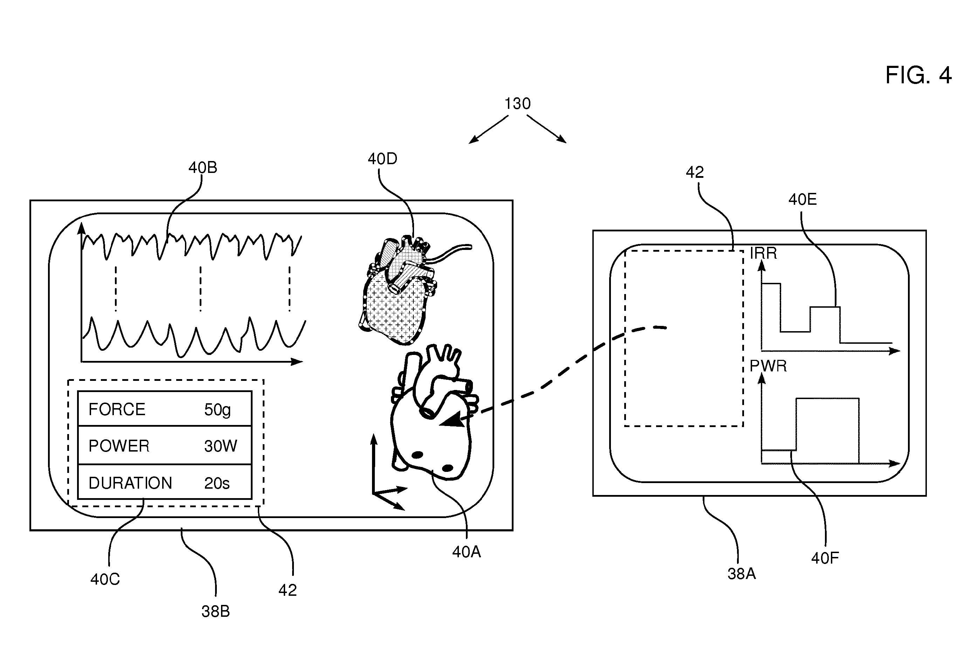

Fig. 4 illustrates the generation of a new spatial relationship 130 of displayed elements using flowchart 90, according to an embodiment of the present invention. Fig. 4 assumes that initial spatial relationship 60 of elements 40, for a given sub-state of a given medical procedure, is as illustrated in Fig. 2, so that Fig. 2 corresponds to step 100 of the flowchart 90. Using steps 112 - 120 of the flowchart, professional 18 generates new spatial arrangement 130 of elements 40, by using gaze direction to relocate movable element 40A from a location on monitor 38A to a new location on monitor 38B.

The flowchart of Fig. 3 has been described assuming that a particular professional performs a given medical procedure. The flowchart may be adapted, *mutatis mutandis,* to accommodate the particular professional performing different medical procedures. In this case the initial spatial relationship of elements 40 is typically different for each procedure.

In some cases, even for the same medical procedure, different initial spatial relationships may be presented on monitors 38, according to requirements of different respective professionals performing the procedure. The flowchart of Fig. 3 may also be adapted to accommodate this situation.

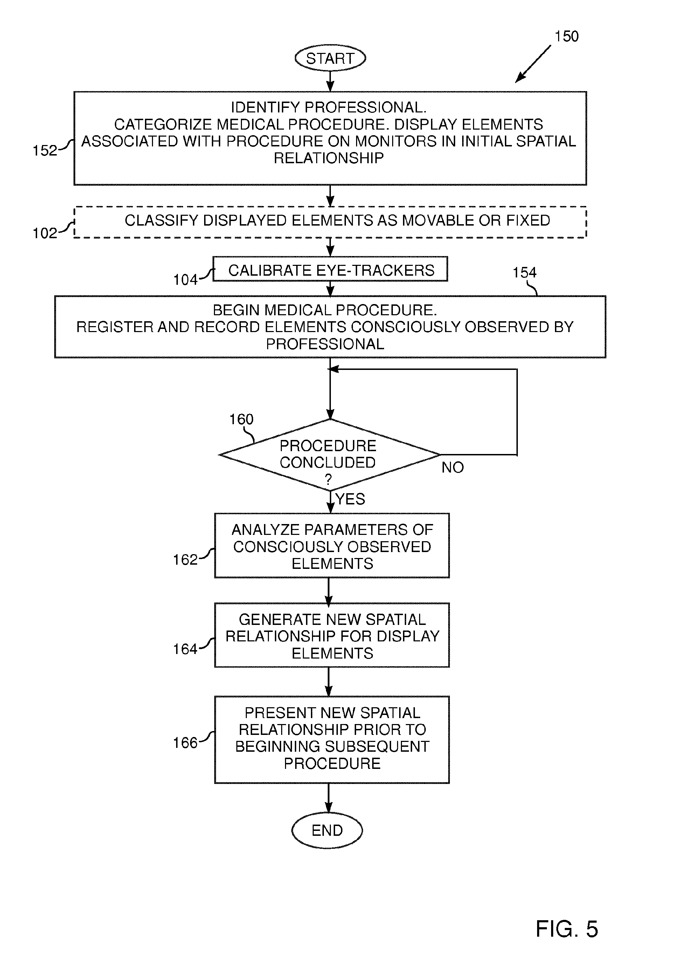

Fig. 5 is a flowchart 150 describing steps taken during operation of system 10, according to an alternative embodiment of the present invention. Flowchart 150 is assumed to be applicable in the case where system 10 comprises two or more monitors 38. Apart from the differences described below, operations generated by flowchart 150 are generally similar to those generated by flowchart 90 (Fig. 2), and steps indicated by the same reference numerals in both flowcharts have generally similar actions.

An initial step 152 is generally similar to step 100 of flowchart 90, except that there is no definition of sub-states. In some embodiments element classification step 102 is optional, i.e., elements 40 may not be classified as movable or fixed. In flowchart 150 step 102 is indicated as optional by the broken lines of the rectangle.

A register and record consciously observed element step 154 is generally the same as step 106, except that sub-states are not registered or recorded.

In step 154 the registration and recording of elements 40 which are consciously observed continues until the procedure being performed concludes. The conclusion of the procedure is checked by a comparison step 160.

Once the procedure has concluded, in an analysis step 162 processor 22 analyzes elements 40 to determine which elements have been consciously observed during the procedure. Typically the analysis comprises tabulating the number of times, or the overall length of time, that a given element 40 is consciously observed.

In a new spatial generation step 164, the processor determines which elements have been observed the most frequently, and groups these elements together for presentation on monitors 38. Typically the grouping relocates the most frequently observed elements onto a single monitor 38.

In a final spatial presentation step 166, the processor presents the new spatial presentation, on the two or more monitors 38, to professional 18. The presentation may be at the conclusion of the procedure begun in step 106. Alternatively, the presentation may be prior to the professional beginning a subsequent procedure that is substantially similar to the procedure of step 106. Typically, in either case, the professional has the option of accepting the new spatial relationship or reverting to the initial spatial relationship.

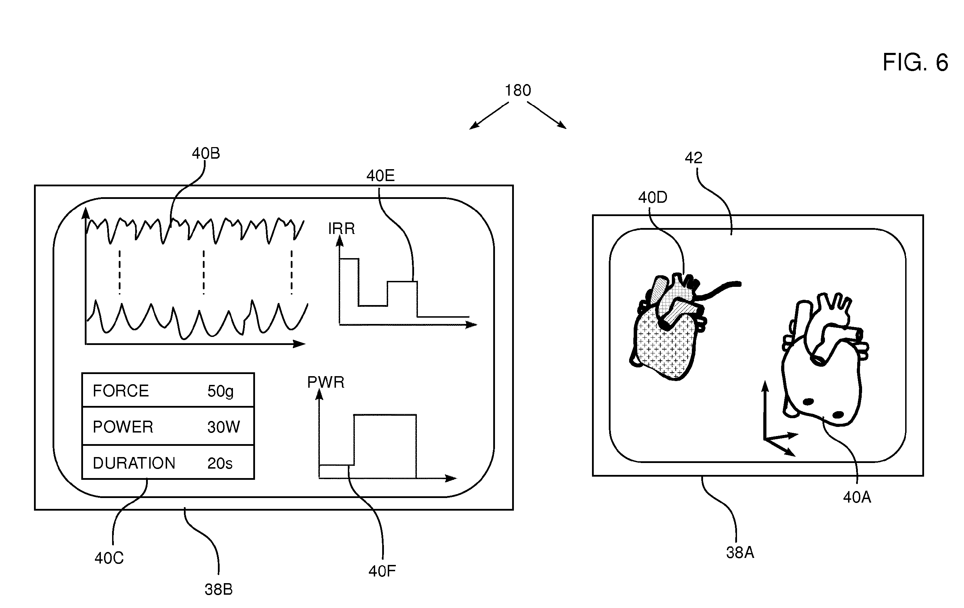

Fig. 6 illustrates presentation of a new spatial relationship 180 of displayed elements, according to an embodiment of the present invention. Relationship 180 is assumed to be presented as described in step 166 of flowchart 150 (Fig. 5). In step 152 spatial relationship 60 (Fig. 2) is displayed, and in analysis step 162 the processor determines that elements 40B, 40C, 40E, and 40F have been most frequently observed, whereas elements 40A and 40D have been less frequently observed. In generation step 164 and presentation step 166 the processor groups the most frequently observed elements (elements 40B, 40C, 40E, and 40F) together, and presents the new grouping on monitors 38. In this example elements 40B, 40C, 40E, and 40F are displayed on single monitor 38B.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A method for displaying information, comprising:
presenting on one or more monitors a plurality of distinct elements in a first spatial relationship with one another, the plurality of distinct elements being illustrative of a state of a medical procedure;
measuring gaze directions, towards the plurality of the elements, of an implementer of the medical procedure while the procedure is being performed; and
in response to the measured gaze directions, rearranging on the one or more monitors the plurality of distinct elements into a second spatial relationship with one another.

2. The method according to claim 1, and further comprising presenting the second spatial relationship on the one or more monitors prior to a subsequent medical procedure being performed by the implementer.

3. The method according to claim 1, and further comprising classifying the distinct elements as movable or fixed distinct elements, and wherein rearranging the plurality of distinct elements comprises relocating at least one of the movable elements while maintaining locations of the fixed elements on the one or more monitors.

4. The method according to claim 1, wherein measuring the gaze directions towards the plurality of the elements comprises registering conscious observation of at least one of the elements.

5. Apparatus for displaying information, comprising:
one or more monitors configured to present a plurality of distinct elements in a first spatial relationship with one another, the plurality of distinct elements being illustrative of a state of a medical procedure;
an eye tracker, configured to measure gaze directions towards the plurality of the elements of an implementer of the medical procedure while the procedure is being performed; and
a processor which is configured, in response to the measured gaze directions, to rearrange on the one or more monitors the plurality of distinct elements into a second spatial relationship with one another.

6. The method according to claim 1 or the apparatus according to claim 5, wherein the one or more monitors comprise two or more monitors.

7. The method according to claim 1 or the apparatus according to claim 5, wherein rearranging the plurality of distinct elements comprises the implementer selecting, using gaze direction, a given distinct element, and further selecting, using gaze direction, a new location for the given distinct element on the one or more monitors.

8. The apparatus according to claim 5, wherein the processor is configured to present the second spatial relationship on the one or more monitors prior to a subsequent medical procedure being performed by the implementer.

9. The apparatus according to claim 5, wherein the processor is configured to classify the distinct elements as movable or fixed distinct elements, and wherein rearranging the plurality of distinct elements comprises relocating at least one of the movable elements while maintaining locations of the fixed elements on the one or more monitors.

10. The method according to claim 1 or the apparatus according to claim 5, wherein the state of the medical procedure comprises a multiplicity of sub-states of the medical procedure, and wherein presenting the plurality of distinct elements comprises presenting the elements during a given sub-state, and wherein measuring the gaze directions comprises measuring the gaze directions during the given sub-state, and wherein rearranging the plurality of distinct elements comprises rearranging the elements for the given sub-state.

11. The method according to claim 10, and comprising defining the sub-states in response to parameters of equipment used during the medical procedure.

12. The apparatus according to claim 10, wherein the processor is configured to define the sub-states in response to parameters of equipment used during the medical procedure.

13. The apparatus according to claim 5, wherein measuring the gaze directions towards the plurality of the elements comprises the processor being configured to register conscious observation of at least one of the elements.

14. The method according to claim 4 or the apparatus according to claim 13, wherein registering conscious observation of the at least one of the elements comprises determining that a time period of a gaze direction towards the at least one of the elements exceeds a preset time.

15. The method according to claim 4 or the apparatus according to claim 13, wherein registering conscious observation of the at least one of the elements comprises determining that a number of observations of the at least one of the elements exceeds a preset number.
